# EUROPEAN PATENT SPECIFICATION

(11) **EP 0 880 941 B1**
(45) Date of publication and mention of the grant of the patent: **19.12.2001**
(21) Application number: 98109843.7
(22) Date of filing: 29.05.1998
(51) Int. Cl.: A61B 18/20

(54) **Laser treatment apparatus**
Laserbehandlungsvorrichtung
Dispositif de traitement par laser

(30) Priority: 30.05.1997 JP 15769497; 30.06.1997 JP 19068697; 29.08.1997 JP 24939697; 29.08.1997 JP 24939797
(43) Date of publication of application: 02.12.1998
(60) Divisional application: 01116584.2
(73) Proprietor: Nidek Co., Ltd., Gamagori-shi, Aichi 443-0035 (JP)
(72) Inventor: Mukai, Hideo, Toyohashi-shi, Aichi, 441-8087 (JP); Ota, Yasuo, Gamagori-shi, Aichi, 443-0104 (JP)
(74) Representative: Prüfer, Lutz H., Dipl.-Phys.

(56) References cited:
- EP-A- 0 075 860
- EP-A- 0 209 992
- WO-A-97/17903
- DE-A- 4 015 066
- DE-A- 4 232 915
- FR-A- 2 622 426
- US-A- 5 350 374

## Description

The present invention relates to a laser treatment apparatus which is used to treat a patient by irradiating a laser beam to a part of body to be treated (diseased part).

Laser treatment apparatus have been used for the treatment of various diseases based on the selection of the wavelength of treating laser. In the recent neoplasty for removing wrinkles, ephelides, birthmarks, stains, etc. of patients, attention is paid to laser treatment apparatus that use the CO₂ laser (carbon dioxide gas laser) having infrared wavelengths. In the neoplasty based on these laser treatment apparatus, a scanning area of a certain shape and size which match with a particular diseased part is selected from among the shapes (square, etc.) and sizes registered in the apparatus, and a treating laser beam is irradiated to the diseased part by deflecting the laser beam to scan the scanning area.

However, diseased parts of ephelides or the like which undergo the neoplasty have a variety of shapes, and therefore the variety of registered shapes of scanning area is insufficient occasionally. Using a larger scanning area so as to include the whole diseased part will risk the increased irradiation of laser to the normal part outside the diseased part. Using a smaller scanning area so as to avoid the irradiation to the normal part will result in a degraded operational efficiency.

The laser treatment apparatus based on the scanning irradiation of a treating laser beam is designed to implement one laser scanning operation in response to a trigger signal from a foot switch or the like operated by the operator. However, more than one laser scanning operation is required depending on each diseased part and its condition, which imposes a degraded operational efficiency and tedious work on the operator.

A laser treatment apparatus according to the preamble of claim 1 is known from EP 0 075 860 A2.

US 5,350,374 discloses the use of a photoelectrical image generating means such as a CCD device for generating an image at a laser irradiated area.

The present invention is intended to deal with the foregoing prior art deficiency, and its prime object is to provide a laser treatment apparatus which enables the appropriate treatment based on the establishment of an optimal laser scanning area.

Another object of the present invention is to provide a laser unit which is capable of performing multiple laser scanning operations efficiently.

The objects are achieved by a laser treatment apparatus according to claim 1.

Further developments of the present invention are given in the dependent claims.

The accompanying drawings, which are incorporated in and constitute a part of this specification illustrate embodiments of the invention and, together with the description, serve to explain the objects, advantages and principles of the invention.

In the drawings,
Fig. 1 is a schematic diagram of the laser treatment apparatus based on an embodiment of this invention;
Fig. 2 is a block diagram showing the basic overall arrangement and the control system of this laser treatment apparatus;
Fig. 3 is a diagram used to explain the hand piece unit of the apparatus based on a first embodiment;
Fig. 4 is a top view of the control panel of this laser treatment apparatus;
Fig. 5 is a diagram used to explain the position of the aligning light spot;
Fig. 6 is a graph showing the variation of the spot position evaluated in terms of voltage signals provided by the x/y axes drive motors against the number of times of operation;
Fig. 7 is a timing chart used to explain the relation among the signals in scan mode;
Fig. 8 is a flowchart used to explain the control of laser scanning operation;
Fig. 9 is a diagram used to explain the hand piece unit of the laser treatment apparatus based on a second embodiment;
Fig. 10 is a diagram used to explain the detection of a marked area;
Fig. 11 is a graph showing the variation of the received light level during the scanning of laser spot on a certain scanning line;
Fig. 12 is a diagram used to explain the hand piece unit of the laser treatment apparatus based on a third embodiment; and
Fig. 13 is a diagram showing an example of display on the display screen at the reading of scanning area.

Embodiments of this invention will be explained with reference to the drawings.

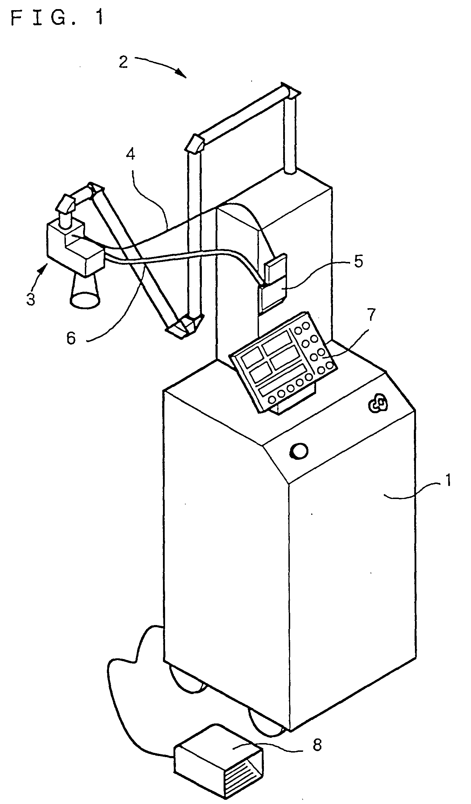

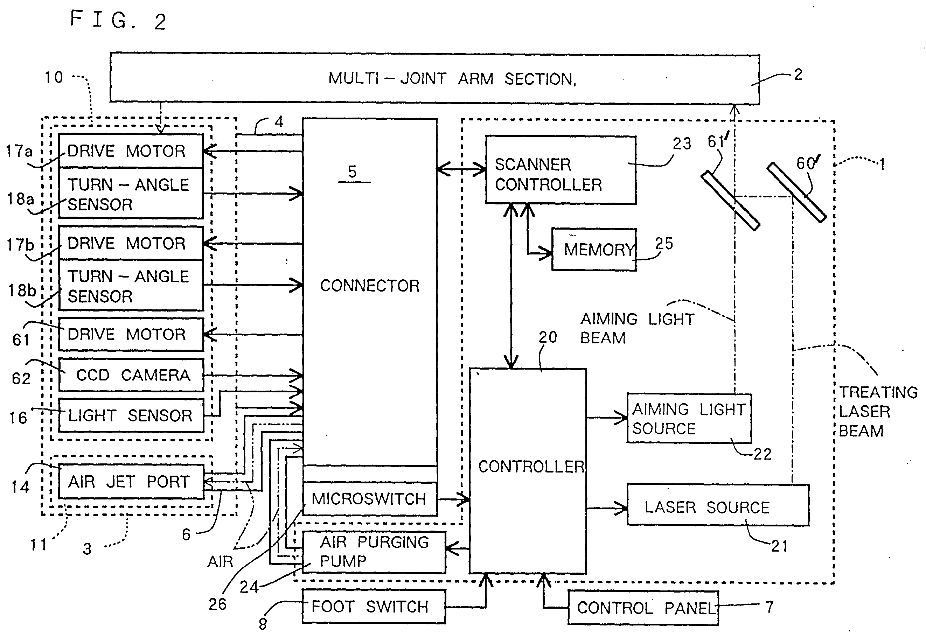

Fig. 1 shows the external view of the laser treatment apparatus based on the first embodiment of this invention. Fig. 2 shows the basic overall arrangement and the control system of the inventive laser treatment apparatus, in which a light sensor 16 is used only in the second embodiment and a drive motor 61 and CCD camera 62 are used only in the third embodiment explained later.

The apparatus includes a laser unit 1, which incorporates a controller 20, a treating laser source 21, an aiming light source 22, a scanner controller 23, an air purging pump 24, and an optical system. In this embodiment, the treating laser source 21 is a CO₂ laser which emits an infrared laser beam, and the aiming light source 22 is a semiconductor laser which emits a visible red laser beam.

The apparatus further includes a multi-joint arm section 2, a hand piece unit 3 which operates to scan a treatment area, a cable 4 and connector 5 with the provision of a microswitch 26 for detecting the connection or disconnection of the cable 4, an air tube 6, a control panel 7 which is used by the operator to set laser irradiation conditions, etc., and a foot switch 8 which is depressed by the operator to produce a trigger signal.

The multi-joint arm section 2 is made up of several rigid pipes linked by joints, so that the operator can move in three-dimensional manner the hand piece unit 3 which is attached at the end of the arm section 2. Mirrors are fitted in the joints of the multi-joint arm section 2, so that the treating laser beam and aiming light beam that are aligned coaxially with each other in the laser unit 1 are guided to pass through the multi-joint arm section 2 and reach the hand piece unit 3.

The hand piece unit 3 is among a variety of detatchable units designed to meet individual purposes and usage. For the following explanation of this embodiment, the apparatus is equipped with a scanning hand piece unit 3 having a scanner head which deflects the laser beam to scan the scanning area of treatment.

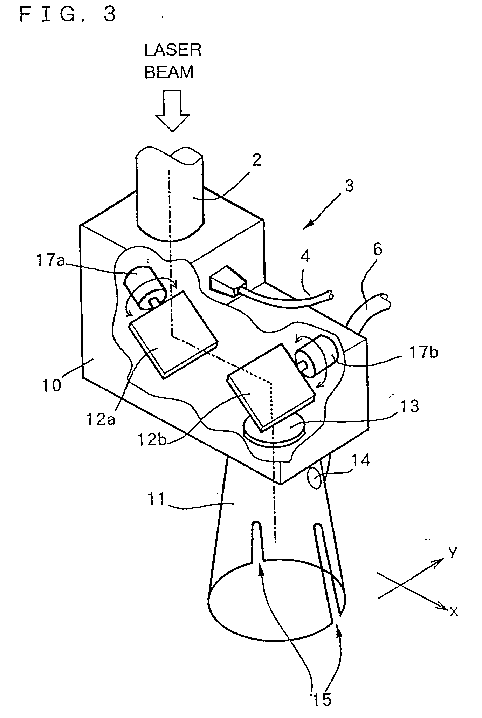

Fig. 3 shows the structure of the scanning hand piece unit 3. It consists of a scanner section 10 and a hand-piece head section 11 which are separable. The scanner section 10 incorporates driven mirrors 12a and 12b which deflect the treating laser beam and aiming light beam, which have come through the multi-joint arm section 2, to scan the treatment area in two-dimensional manner in the x/y directions, drive motors 17a and 17b which swing the mirrors 12a and 12b, and a focusing lens 13 which focuses the treating laser beam to the treatment area.

The drive motors 17a and 17b are controlled by the scanner controller 23 in the laser unit 1. The scanner controller 23 produces drive currents, which are fed through the connector 5 and cable 4 to the drive motors 17a and 17b in the scanner section 10, by which the driven mirrors 12a and 12b are swung.

The hand-piece head section 11 having a generally cone shape to cover the laser scanning area at its bottom is made of transparent resin, so that the operator can view from outside the treatment area and irradiation area covered by the hand-piece head section 11. The operator can bring the bottom of hand-piece head section 11 in stable contact with the patient body and can operate it easily in defining a scanning area of an intended shape, as will be explained later. The hand-piece head section 11 has a vertical dimension so that the treating laser beam is focused exactly to the treatment area by the focusing lens 13.

The hand-piece head section 11 has in its side wall the formation of cuts for fume outlets 15 which clear the treatment area of fumes created by the laser irradiation. In operation, a jet of air produced by the air purging pump 24 in the laser unit 1 is conducted through the connector 5 and air tube 6 to an air jet port 14 located near the top of the hand-piece head section 11, so that an air jet introduced into the cone hood dispels fumes through the fume outlets 15. Incidentally, the bottom of hand-piece head section 11 has the size which can secure the scanning area of the laser beam for the treatment.

Instead of the above-mentioned hand-piece head section of generally cone-shaped transparent resin, a metallic device having at least three poles for the stable placement and the determination of focal distance may be used. Instead of the two fume outlets 15 formed in the side wall of the hand-piece head section 11, more than two fume outlets may be provided.

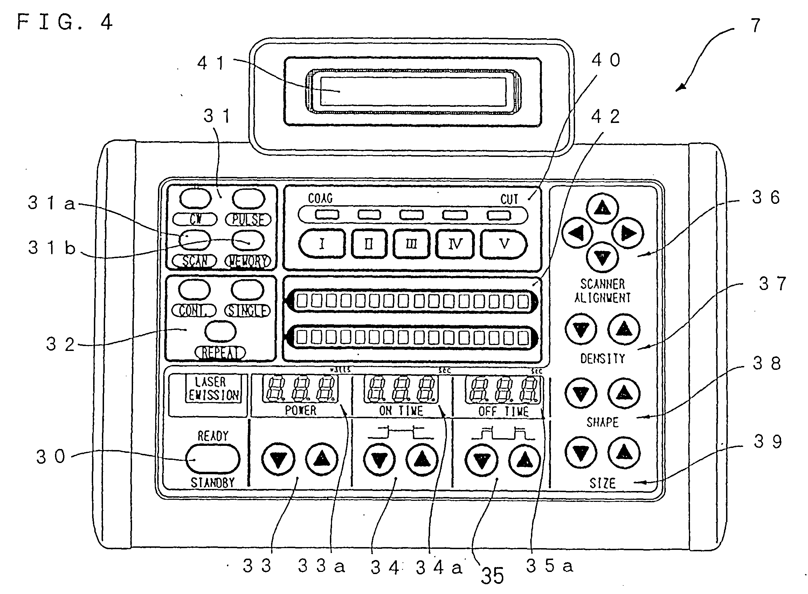

Fig. 4 shows the control panel 7, which includes READY/STANDBY key 30, mode select keys 31, irradiation pattern select keys 32, irradiation power setting keys 33, on-time setting keys 34, off-time setting keys 35, alignment keys 36, irradiation density setting keys 37, irradiation form select keys 38, irradiation sizes select keys 39, pulse interval select keys 40, a display window 41 for displaying information about the scanner, and another display window 42 for displaying error messages and machine states.

Among these keys, the READY/STANDBY key 30 is used by the operator to switch between the ready (laser irradiation enabled) state and the standby (wait) state. In the ready state, the apparatus can irradiate a laser beam in response to the trigger signal from the foot switch 8, whereas in the standby state, the trigger signal is locked so as to preclude the laser irradiation caused by the erroneous input of the trigger signal at the setting of conditions. In memory mode, which will be explained later, the READY/STANDBY key 30 also functions as START key and ENTER key in defining a scanning area of intended shape.

The mode select keys 31 are used by the operator to designate the CW mode for continuous emission, pulse mode for pulsative emission, scan mode, or memory mode. Indicated by 31a is scan mode select key, and 31b is memory mode select key. When the operator selects a mode, the sign below the relevant key lights up to indicate the established mode. In scan mode, a treating laser beam is irradiated to scan the scanning area at a pulse oscillation frequency of 300 Hz.

The irradiation pattern select keys 32 are used to designate the CONT (continuous irradiation) pattern in the CW mode or pulse mode, the single (one-shot irradiation) pattern and the repeat (repetitive irradiation) pattern in the CW mode, pulse mode or scan mode. When the operator selects a pattern, the sign below the relevant key lights up to indicate the selected pattern.

In CW mode or pulse mode, if the single pattern is selected, laser irradiation for the duration set by the on-time setting keys 34 takes place once, or if the repeat pattern is selected, laser irradiation cycles including an exposure period of the duration set by the on-time setting keys 34 and a halt period set by the off-time setting key 35 take place repeatedly on condition that the trigger signal from the foot switch 8 is active. The single pattern and repeat pattern in scan mode will be explained in detail later.

The irradiation power setting keys 33 including UP key and DOWN key are used to set the power level of the treating laser beam, with the setup power level being displayed in the associated display window 33a. The range of setting is from 0.1 to 40 W in the case of CW mode (the maximum value differs depending on the mode) in 0.1 W step in the 0.1-1 W range and in 1 W step in the 1-40 W range.

The on-time setting keys 34 including UP key and DOWN key are used for the operation with the single pattern or repeat pattern in CW mode or pulse mode to set the duration of laser irradiation, with the setup duration being displayed in the associated display window 34a. The range of setting is from 0.02 to 5 sec. in this embodiment in 0.02 sec. step in the 0.02 - 0.1 sec. range, in 0.1 sec. step in the 0.1 - 1 sec. range and in 1 sec. step in the 1 - 5 sec. range.

The off-time setting keys 35 including UP key and DOWN key are used for the operation with the repeat pattern in CW mode, pulse mode or scan mode to set the duration of halt of laser irradiation, with the setup duration being displayed in the associated display window 35a. The range and step of setting are identical to those of the on-time setting keys 34.

The alignment setting keys 36 are used for the fine adjustment of irradiation position in scan mode and the movement of laser spot in memory mode. For the fine adjustment of irradiation position in scan mode, the alignment keys 36 allow the operator to move the position of irradiation area without the need of moving the hand-piece head section 11.

The irradiation density setting keys 37 including UP key and DOWN key are used to set the laser irradiation density in scan mode, with the setup density being displayed in the display window 41.

The irradiation form select keys 38 including UP key and DOWN key are used in scan mode to select a shape of laser irradiation area (and, at the same time, the irradiation area of the aiming light) from among the preset triangle, square, hexagon, etc. and an arbitrary shape registered by the operator in memory mode. The selected form is displayed in the display window 41. These keys 38 are used in memory mode to register an arbitrary shape of scanning area and select a stored location (file) in the memory.

The irradiation size select keys 39 including UP key and DOWN key are used in scan mode to select the size of laser irradiation area (and, at the same time, the irradiation area of the aiming light). The selected size is displayed in the display window 41.

The pulse interval select keys 40 are used in pulse mode to select a laser pulse irradiation interval from among five values.

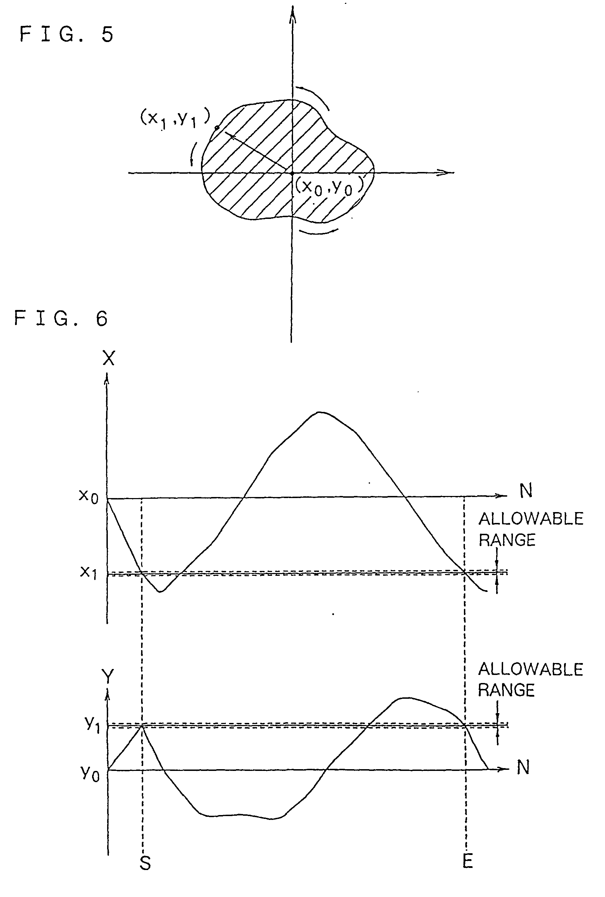

Next, the operation of the laser treatment apparatus arranged as described above for defining an intended irradiation area in memory mode will be explained with reference to Fig. 5 which explains the position of aligning light spot and Fig. 6 which shows the variation of the spot position evaluated in terms of voltage signals provided by the x/y axes drive motors for the driven mirrors 12a and 12b against the number of times N of operation.

When the apparatus is turned on, the controller 20 implements the diagnostic test for each section. On completion of the tests, the system automatically enters the standby state, causing the control panel 7 to light up the STANDBY sign below the key 30 and display a message of "standby state" in the display window 42. The system is initialized following the power-on event to designate automatically CW mode and CONT pattern, with the CW and CONT signs lighting up on the control panel 7.

The apparatus checks the connection of the cable 4 to the connector 5 by means of a microswitch 26 in the diagnostic test (or during the power-on period), and if disconnection is detected, the controller 20 cuts off the power supply to the scanner controller 23, thereby preventing the malfunctioning of the apparatus.

The operator confirms the system in standby state by viewing the display on the control panel 7, and brings the entire bottom plane of the hand-piece head section 11 in contact with the patient's body so that the portion to be treated is located at the center of the bottom plane. This handling manner of hand-piece head section 11 enables to put the hand piece unit 3 stably on the patient's body, facilitating the operations in defining a scanning area.

For defining a scanning area which meets the shape of treatment area, the operator pushes the memory mode select key 31b on the control panel 7, and the system enters the memory mode. Receiving the signal from the key 31b, the controller 20 lights up the MEMORY sign on the control panel 7 and displays file information such as the storage file names on the display window 41 so that the operator can select one of multiple storage files in the memory 25 by using the irradiation form select keys 38.

The memory mode select signal is sent to the scanner controller 23 by way of the controller 20. The scanner controller 23 responds to the memory mode select signal to control the drive motors 17a and 17b thereby to return the driven mirrors 12a and 12b to their home positions. The position of the aligning light spot, which is the same as the spot of aiming light beam in this embodiment, at this time is expressed in terms of x/y coordinates to be (x,y)=(x₀,y₀).

The operator who has confirmed the system in memory mode uses the alignment keys 36 to move the aligning light spot from the initial position (x₀,y₀) to the outer bound of the treatment area (shown by hatching in Fig. 5). The spot moving signal from the alignment keys 36 is sent by way of the controller 20 to the scanner controller 23, which operates on the drive motors 17a and 17b to swing the driven mirrors 12a and 12b, thereby moving the aligning light spot. If the aligning light spot reaches the limit of movement, it is indicated to the operator by means of a buzzer (not shown) or by a message on the display window 41.

Upon knowing the arrival of the aligning light spot at the outer bound of the treatment area, the operator pushes the READY/STANDBY key 30 to begin the definition of a scanning area. The position of the aligning light spot is recognized in terms of voltage signals produced by turn-angle sensors 18a and 18b attached to the drive motors 17a and 17b (or driven mirrors 12a and 12b).

The scanner controller 23 memorizes in the memory 25 the position (x₁,y₁) of aligning light spot (indicated by S in Fig. 6) of the time of pushing of the READY/STANDBY key 30, i.e., at the starting of the definition of scanning area. The system can be designed to display the starting position and current position of the aligning light spot on the display window 41 so that the - operator can recognize briefly the starting position in relation with the current position.

Subsequently, the operators uses the alignment keys 36 to move the aligning light spot along the outer bound of the treatment area. The scanner controller 23 stores the voltage signals, which vary with the movement of the aligning light spot, in the memory 25 at every count of a certain number of times of operation of the alignment keys 36, or alternatively at a certain time interval.

The operator moves the aligning light spot from the starting position (x₁,y₁) to go around the treatment area toward the starting position. The scanner controller 23 compares the voltage signals to be stored in the memory 25 with the voltage signals of the starting position (x₁,y₁), and when the voltage signals of the x and y axes both enter the allowable range of the starting position (indicated by E in Fig. 6), it activates the buzzer.

Upon hearing the buzzer signal, the operator pushes the READY/STANDBY key 30 to establish the scanning area. The scanner controller 23 responds to the signal of key 30 to figure the scanning area by carrying out the computation of interpolation (any of the known interpolation schemes) based on the data of position signals stored along the outer bound of treatment area from the starting position to the ending position.

The scanner controller 23 responds to the pushing of the READY/STANDBY key 30 to trace with the aligning light spot the outer bound of the scanning area stored in the memory 25 so that the operator visually recognizes the scanning area. The operator is prompted by a message on the display window 41 as to the approval or cancellation of the defined scanning area. The operator pushes the scan mode select key 31a or memory mode select key 31b to approve or cancel the displayed scanning area. The system switches to scan mode in response to the signal of scan mode select key 31a, or returns the aligning light spot to the initial position (x₀,y₀) for defining another scanning area in response to the signal of memory mode select key 31b.

Following the switching to scan mode by the pushing of the scan mode select key 31a, the operator carries out various settings, such as of irradiation conditions, and the fine adjustment of irradiation position by using the alignment keys 36. Subsequently, the operator pushes the READY/STANDBY key 30 so that the system turns to the ready state. Next, the operator depresses the foot switch 8 to generate a trigger signal.

The controller 20 which has received the trigger signal operates on the laser source 21 to emit a treating laser beam in accordance with the settings on the control panel 7. The treating laser beam from the laser source 21 is reflected by a mirror 60' and dichroic mirror 61', aligned coaxially to the aiming light beam from the aiming light source 22, and introduced to the multi-joint arm section 2. The coaxial treating laser beam and aiming light beam are reflected by the mirrors located at the joints of the arm section 2 and led to the hand piece unit 3.

The trigger signal from the foot switch 8 is sent through the controller 20 to the scanner controller 23, which operates on the drive motors 17a and 17b in accordance with the setup signals of the form and size of irradiation area, etc. thereby to deflect the treating laser beam to scan the treatment area. During the irradiation of the treating laser beam, the controller 20 activates the air purging pump 24 to feed air into the hand-piece head section 11, thereby expelling fumes created by laser irradiation through the fume outlets 15.

In case the single pattern for one scanning operation is selected, the controller 20 and scanner controller 23 detects the end of scanning by receiving the feedback signal from the hand piece unit 3. Based on the scanning end signal, the controller 20 operates on a shutter (not shown) to interrupt the laser beam from the laser source 21, while the scanner controller 23 operates on the drive motors 17a and 17b so that the aiming light beam traces the outer bound of scanning area.

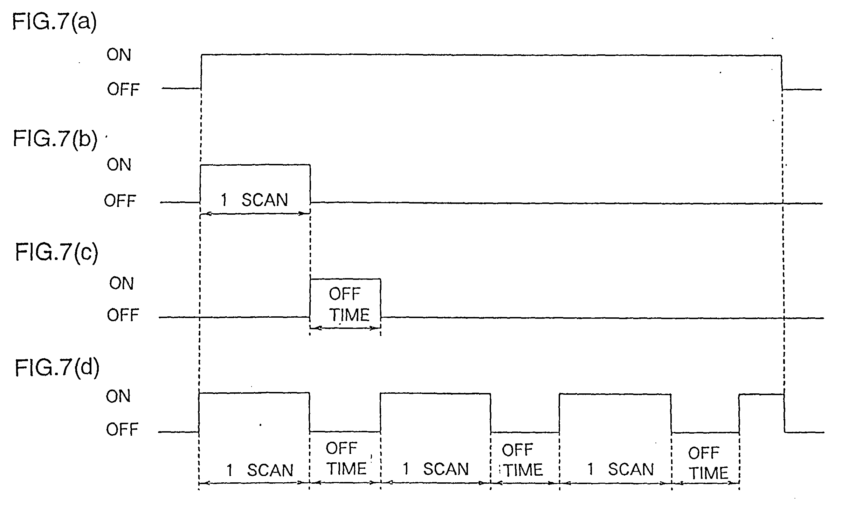

With the single pattern being selected, even if the trigger signal from the foot switch 8 retains active as shown by (a) in Fig. 7, the laser irradiation and scanning operation terminate on completion of one scanning operation as shown by (b) in Fig. 7.

Next, the repetitive scanning operation which takes place by the selection of the repeat pattern will be explained.

When the operator keeps the foot switch 8 depressed, it produces the trigger signal continuously as shown by (a) in Fig. 7. The trigger signal is received by the controller 20 and scanner controller 23, which then implement the laser irradiation of one scanning in the same manner as the case of the single pattern. On completion of one-scanning laser irradiation, the scanner controller 23 detects the end of scanning and sends the end signal to the controller 20.

The controller 20 interrupts the laser beam from the laser source 21, and operates its repeat circuit (not shown) to turn on the OFF signal as shown by (c) in Fig. 7 thereby to invalidate the trigger signal. On expiration of the off-time which has been set with the off-time setting key 35, the repeat circuit in the controller 20 turns off the OFF signal thereby to validate the trigger signal.

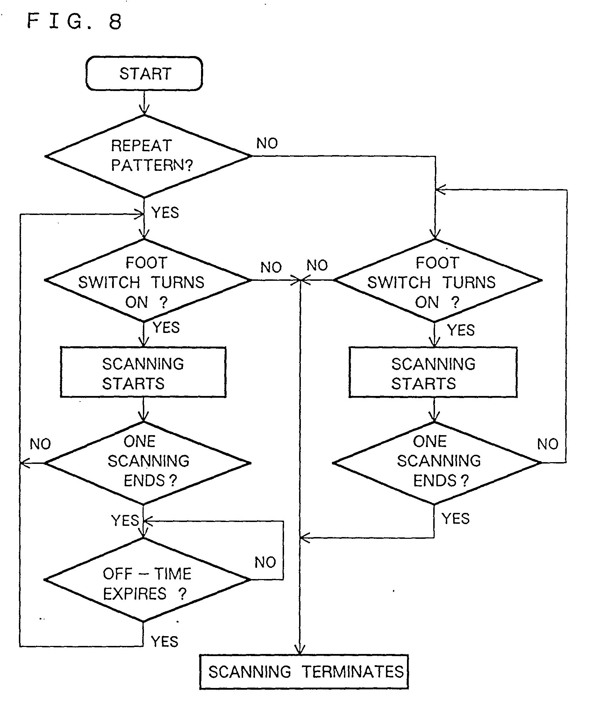

In response to the turn-off of the OFF signal, the controller 20 and scanner controller 23 receive the trigger signal from the foot switch 8 again, and restart the laser irradiation and scanning operation. These operations of laser beam scanning irradiation are repeated as long as the scanner controller 23 receives the trigger signal from the foot switch 8 as shown by (a) and (d) in Fig. 7. When the trigger signal ceases, the laser beam scanning irradiation halts (the laser irradiation halts immediately even before the end of scanning operation). The foregoing operations are shown by the flowchart of Fig. 8.

As described above, the operator can operate the apparatus with the repeat pattern, i.e., in repeat mode, to carry out easily the laser beam scanning irradiation an intended number of times, while depressing the foot switch 8 continuously. The operator is allowed to observe the progress of treatment for every scanning operation based on the setting of an intended off-time.

Although in this embodiment the alignment keys 36 on the control panel 7 are used to move the aligning light spot in defining a scanning area of an intended shape, the use of other input device such as the joystick, mouse or trackball in place of the key set 36 will improve the operability for the movement of aligning light spot.

Although in this embodiment positional data is stored at every count of a certain number of times of key operation (or at a certain time interval) in defining a scanning area, an alternative system design is the provision of a data input key so that the operator pushes the key to store data of every particular position.

Although in this embodiment a scanning area is defined based on the presetting of the starting position, an alternative system operation is to allow the continuous storing of positional data following the switching to memory mode and detect the complete cycle along the outer bound of scanning area in terms of x/y coordinates.

Next, the laser treatment apparatus based on a second embodiment will be explained. The external view overall arrangement and control system of this laser treatment apparatus are basically identical to those of the preceding first embodiment, with the common reference symbols being used, and explanation thereof will be omitted.

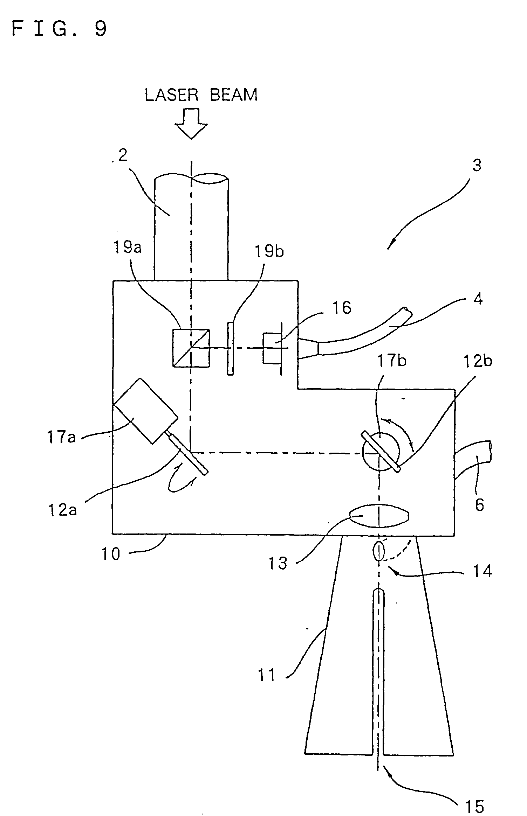

The second embodiment shown in Figs. 9 to 11 is as far as it relates, to the detection of the reflection light level with a light sensor not an embodiment of the invention.

Fig. 9 shows the structure of the scanning hand piece unit 3 based on this embodiment, which includes portions identical to those of the first embodiment, with their explanation being omitted.

The hand piece unit 3 has its scanner section 10 incorporating driven mirrors 12a and 12b, their associated drive motors 17a and 17b, a focusing lens 13, a beam splitter 19a, a filter 19b, and a light sensor 16. The filter 19b has the optical characteristics for blocking the infrared light and transmitting the visible light (the aiming light beam reflected on the patient's skin) so that the infrared light beam is prevented from entering the light sensor 16.

The control panel 7 and the key layout and functions are basically identical to the preceding first embodiment, except that the alignment keys 36 are used for setting a threshold value, which will be explained later, in memory mode.

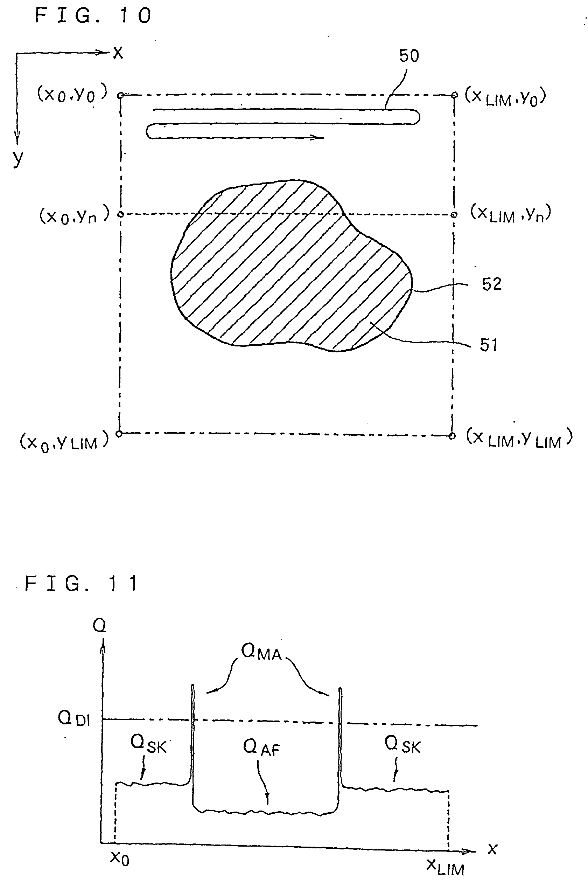

The following explains with reference to Fig. 10 and Fig. 11 the operation of the apparatus having the above-mentioned hand piece unit 3 in implementing the laser irradiation to a scanning form which has been established arbitrarily by the operator. Fig. 10 explains the detection of a marked area, and Fig. 11 shows the variation of the light level detected by the light sensor 16 during the scanning of laser spot on a certain scanning line.

The treatment area 51 has its outer bound marked in advance in a color that is highly reflective, e.g., fluorescent color, for the wavelength of the light of aiming light source 22. This embodiment bases the setting of a scanning area having a marked profile 52 on the scanning illumination of the patient' s skin with the aligning light spot, i.e., spot of aiming light beam, and the detection of the reflection light level with the light sensor 16, and therefore a marking color that is distinct from the skin color of the patient is used preferably.

Subsequently, the operator pushes the memory mode select key 31b on the control panel 7 to turn the system into memory mode for storing the scanning form of laser irradiation. Storage file names are displayed on the display window 41, and the operator selects a file name by using the irradiation form select keys 38.

In response to the switching to memory mode, the scanner controller 23 operates on the drive motors 17a and 17b to return the driven mirrors 12a and 12b to their home positions of memory mode. In the following explanation, the irradiation position of the treating laser beam and aligning light spot is expressed by x/y coordinates, and the driven mirrors 12a and 12b have their home positions expressed in terms of the beam position to be (x,y)=(x₀,y₀).

With memory mode being selected, the operator selects the high level or low level relative to the threshold light level for the detection of marked profile by using UP or DOWN key of the alignment keys 36. Specifically, in this embodiment, the operator selects the high light level to suit the high-reflective marking.

The operator sets the threshold light level for mark detection by using RIGHT key and LEFT key of the alignment keys 36. The setup value is displayed on the display window 41. For this setting procedure, the reflection light level at the initial position (x₀,y₀) of the aligning light spot is detected and read out on the display window 41 thereby to facilitate the determination of threshold light level by the operator.

Finally, the operator sets the irradiation density by using the irradiation density setting keys 37. A higher irradiation density enables the definition of a more precise scanning area.

Following these settings, the operator puts the bottom of hand-piece head section 11 on the patient's body so that the portion to be treated is located virtually at the center. The operator pushes the READY/STANDBY key 30 to start the reading of the scanning area. Namely, the key 30 functions in memory mode to start the reading of the scanning area.

The scanner controller 23 receives the signal from the key 30 by way of the controller 20 and operates on the drive motors 17a and 17b to control the turn angle of the driven mirrors 12a and 12b so that the aligning light spot moves as shown by the arrow 50 in Fig. 10. The rectangle of 2-dot & dash lines in Fig. 10 indicates the movable range of the aligning light spot achieved by the movement of the driven mirrors.

The aligning light spot scans the x/y plane ranging from X₀ to X_{LIM} and from y₀ to y_{LIM} in a step at the scanning line density set with the keys 37. Specifically, the aligning light spot moves from the origin (x₀,y₀) to the x-axis limit point (x_{LIM},y₀), then turns to the y-axis direction and moves to point (x_{LIM},y₁), and moves along the x axis in the opposite direction to point (x₀,y₁). In this manner, the aligning light spot scans the whole range up to the point (x_{LIM},y_{LIM}).

During the scanning operation, the aiming light beam from the aiming light source 22 goes through the multi-joint arm section 2 and enters the hand piece unit 3, in which the laser beam is reflected by the driven mirrors 12a and 12b and projected to illuminate the patient' s skin. The reflected aiming light beam from the patient's skin comes back into the hand piece unit 3, in which it is reflected by the driven mirrors 12a and 12b and beam splitter 19a and is incident to the light sensor 16 through the filter 19b.

Fig. 11 shows the variation of the received light level when the aligning light spot scans from point (x₀, yₙ) to point (x_{LIM},yₙ). Since the marked profile 52 has higher reflectivity than other normal and diseased parts of the skin, the received light level takes peak values Q_{MA} when the scanning light spot crosses the outer bound in contrast to the normal part and diseased part having low reflection light levels Q_{SK} and Q_{AF}, respectively. Accordingly, it is possible to detect the x-coordinate of the marked profile 52 on the scanning line of yₙ based on a threshold value Q_{DI}.

This process is implemented for the whole y-axis range from y₀ to y_{LIM} to detect the coordinates of the entire marked profile 52, and the interior of this outer bound is determined to be the scanning area. X/y coordinates of the aligning light spot are recognized in terms of voltage signals produced by the turn-angle sensors 18a and 18b of the drive motors 17a and 17b (or driven mirrors 12a and 12b), and stored in the memory 25. It is desirable to introduce the interpolation process and noise data eliminating process in collecting the coordinate data of the marked profile for the determination of scanning area.

After the scanning area is determined, the scanner controller 23 operates on the drive motors 17a and 17b based on the scanning area forming information read out of the memory 25 so that the aligning light spot traces the outer bound of scanning area, thereby allowing the operator to confirm the scanning area of laser irradiation.

The operator is prompted by a message on the display window 41 as to the approval or cancellation of the stored scanning area. The operator pushes the scan mode select key 31a or memory mode select key 31b to approve or cancel the scanning area. In the case of cancellation, the operator modifies the marking of scanning area and runs the system to implement the reading of scanning area in the same procedure as explained above.

The system switches to scan mode in response to the approving signal of scan mode select key 31a, allowing the operator to carry out various settings, such as of irradiation conditions, and the fine adjustment of irradiation position with the alignment keys 36. Subsequently, the operator pushes the READY/STANDBY key 30 so that the system turns to the ready state. Next, the operator depresses the foot switch 8 to generate a trigger signal.

The controller 20 which has received the trigger signal operates on the laser source 21 to emit a treating laser beam in accordance with the settings on the control panel 7. The trigger signal from the foot switch 8 is sent through the controller 20 to the scanner controller 23, which operates on the drive motors 17a and 17b in accordance with information on the stored scanning area so that the treating laser beam is irradiated to scan the treatment area.

Irradiation of laser beam to the established scanning area may be controlled such that the laser beam is turned on and off depending on information on the stored scanning area and in correspondence to the scanning operation which is carried out uninterruptedly by the drive motors 17a and 17b.

Although in the foregoing embodiment the treatment area is marked with a highly reflective profile, marking in a low-reflective color, e.g., black, which provides a light level below the threshold and is clearly distinct from the patient's skin color can be used for the detection of the scanning area. Accordingly, it is possible to choose a proper marking adapted to the patient's skin color, i.e., bright marking for patients having a dark skin and dark marking for patients having a whitish skin, thereby defining and detecting the scanning area more clearly. Marking may be made in the entire treatment area, instead of only the profile of area, for the definition and detection of the treatment area. If the difference in color between the normal part and diseased part is distinct, the scanning area can be defined by itself and can be detected without the need of marking.

Although in this embodiment both driven mirrors 12a and 12b are operated to read the marked profile of area with the aligning light spot, an alternative scheme is the use of a linear CCD or two-dimensional CCD in place of the light sensor 16 and the provision of an illumination optical system which illuminates the entire laser irradiation range virtually uniformly. In case the linear CCD is used, one of the driven mirrors 12a and 12b is operated, and the scanning area is determined from the position detection signal of the CCD and the drive signal of the mirror. In case the two-dimensional CCD is used, the driven mirrors 12a and 12b are left inactive in memory mode, and the scanning area is determined solely from the position detection signal of the CCD. If a CCD camera operative under the usual room illumination is used for the two-dimensional CCD, the provision of illumination optical system will not be required.

Although in this embodiment x/y coordinates of marked profile are detected in terms of turn angles of the driven mirrors 12a and 12b, an alternative scheme is based on the storing of the time length since the start of mark sensing until each recognition of marked profile.

Next, the laser treatment apparatus based on the third embodiment of this invention will be explained. The external view, overall arrangement and control system of this laser treatment apparatus are basically identical to those of the preceding first embodiment, with the common reference symbols being used, and explanation thereof will be omitted.

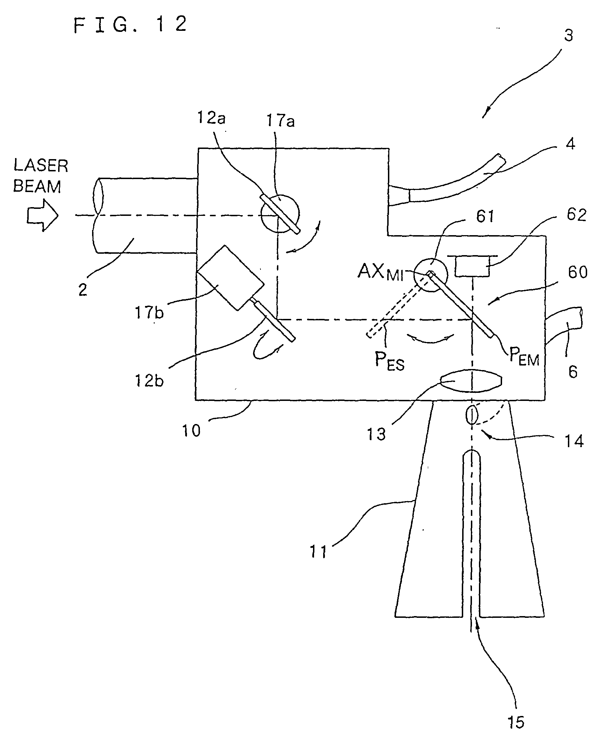

Fig. 12 shows the structure of the scanning hand piece unit 3 based on this embodiment, which includes portions identical to those of the first embodiment, with their explanation being omitted.

The scanner section 10 incorporates driven mirrors 12a and 12b which deflect the treating laser beam and aiming light beam, which have come through the multi-joint arm section 2, to scan the treatment area in the x/y directions, and their associated drive motors 17a and 17b. The laser beam and aiming light beam reflected by the driven mirrors 12a and 12b are further reflected by another driven mirror 60 and irradiated through a focusing lens 13 onto the treatment area.

The scanner section 10 further includes a drive motor 61 for the driven mirror 60, and a two-dimensional CCD 62 having the sensitivity of visible light. The driven mirror 60 is pivoted to swing on the axis AX_{MI}, and it is turned to the position as shown by solid-line figure P_{EM} during the laser irradiation thereby to guide the treating laser beam to the treatment area and, at the same time, prevent a reflected laser beam coming from the treatment area from entering the CCD camera 62. When laser irradiation is not taking place, the driven mirror 60 is kept at the position as shown by dashed-line figure P_{ES}, so that the CCD camera 62 can image the treatment area.

The control panel 7 and the key layout and functions are basically identical to the preceding first embodiment, except that the display window 41 is a LCD touch-panel display screen which displays figures (icons) of keys so that the operator instructs the selection of items by touching respective icons of keys.

The display screen 41 displays the image of diseased part taken by the CCD camera 62. The display screen 41 is designed to compose electronically the scanning form selected with the irradiation form select keys 38 or scanning form defined in memory mode over the image of diseased part, so that the operator can check the scanning area with respect to the diseased part even without using the aiming light beam (with the driven mirror 60 being retracted to the P_{ES} position). The electronic composition of scanning form varies automatically in response to the operation of various setting keys, allowing the operator to confirm the resulting scanning area on the display screen 41.

The display screen 41 further includes icons of INPUT key 41a and READ key 41b used in memory mode. With the INPUT key 41a being turned on, the operator uses a touch pen or similar device (not shown) to trace the outer bound of the displayed diseased part, and then the scanning area is defined and stored in the memory 25. With the READ key 41b being turned on, the scanner controller 23 detects the outer bound of diseased part based on the image taken by the CCD camera 62 and stores the resulting scanning area in the memory 25 automatically.

The display window 42 is used to display the selected mode and pattern, operational instructions, error messages, etc. of the apparatus.

The following explains the operation of the laser treatment apparatus arranged as explained above in implementing the laser irradiation to a scanning form which been established automatically in memory mode.

When the apparatus is turned on, the controller 20 implements the diagnostic test for each section. On completion of the tests, the system automatically enters the standby state, causing the control panel 7 to light up the STANDBY sign below the key 30 and display a message of "standby state" in the display window 42.

Subsequently, the controller 20 operates on the scanner controller 23 to activate the drive motor 61 so that the driven mirror 60 is turned to the retracted position shown by P_{ES}. At this time, the treating laser beam and aiming light beam are interrupted by a shutter (not shown) which is operated by the controller 20.

The operator brings the entire bottom plane of the hand-piece head section 11 in contact with the patient's body so that the portion to be treated is located virtually at the center. The display screen 41 displays the image of diseased part taken by the CCD camera 62, allowing the operator to make easy positioning of the hand piece unit 3, while observing the image of diseased part, with the unit 3 being kept placed in the area of diseased part.

For starting the definition of scanning area, the operator pushes the memory mode select key 31b on the control panel 7, and the system switches to memory mode. Receiving the signal from the key 31b, the controller 20 lights up the MEMORY sign on the control panel 7 and displays file information such as the storage file names on the display screen 41 so that the operator can select a storage file by using the irradiation form select keys 38.

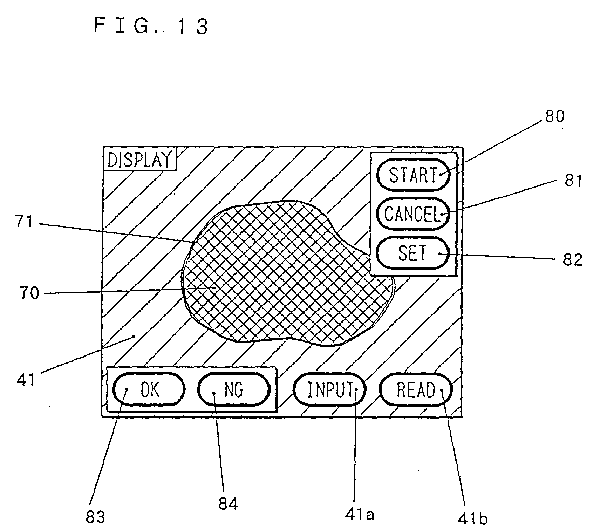

The operator confirms the system in memory mode, and points the INPUT key 41a in the case of drawing a scanning area manually with the touch-pen, or points the READ key 41b in the case of defining a scanning area automatically based on the image data taken by the CCD camera 62. The following explains, in connection with an example of display shown in Fig. 13, the operation of the latter case for defining a scanning area automatically.

When the operator points the READ key 41b, icons of START key 80, CANCEL key 81 and SET key 82 appear in the display screen 41. When the operator points the START key 80, the scanner controller 23 detects the diseased part of patient's body based on the intensity of pixels of the image taken by the CCD camera 62 and defines a scanning area of laser irradiation.

Among a variety of image processing techniques useful for the detection of diseased part, one example is to recognize the outer bound of diseased part by detecting the edge of image based on the spatial differentiation, binary conversion, and profile extraction by utilization of the fact that a diseased part is generally darker than the normal part of the skin. In this case, the threshold value of binary conversion process can be altered with the SET key 82. In case there is little difference in brightness between the diseased part and normal part, the diseased part (or outer bound of it) may be marked to provide a distinct reflectivity.

Following the definition of the scanning area of laser irradiation, the display screen 41 displays the profile image 71 of scanning area over the diseased part image 70 taken by the CCD camera 62. At the same time, icons of approval (OK) key 83 and cancellation (NG) key 84 appear in the display screen.

The operator examines the displayed scanning area, and points the approval key 83 or cancellation key 84. The scanner controller 23 responds to the pointing of approval key 83 to store the information of scanning area in the memory 25. The display screen 41 has its key icons erased, while having the profile image 71 kept displayed over the diseased part image 70. Otherwise, if the cancellation key 84 is pointed, the icons of START key 80, CANCEL key 81 and SET key 82 reappear, allowing the operator to instruct the operation to be carried out. When the CANCEL key 81 is pointed, the initial screen of memory mode is restored.

On completion of the definition of scanning area, the operator pushes the scan mode select key 31a so that the system switches to the scan mode. Subsequently, the operator carries out various settings, such as of irradiation conditions, and the fine adjustment of irradiation position by using the alignment keys 36. Since the diseased part image 70 and profile image 71 can be observed on the display screen 41, while the hand piece unit 3 is kept placed in the area of diseased part, the operator can adjust the irradiation position easily.

Following the setting of irradiation conditions and determination of irradiation area, the operator pushes the READY/STANDBY key 30 so that the system turns to the ready state. Next, the operator depresses the foot switch 8 to generate a trigger signal.

The controller 20 which has received the trigger signal operates on the laser source 21 to emit a treating laser beam in accordance with the settings on the control panel 7. The trigger signal from the foot switch 8 is sent through the controller 20 to the scanner controller 23, which operates on the drive motor 61 to turn the driven mirror 60 to the irradiation position P_{EM} and operates on the drive motors 17a and 17b in accordance with the setting conditions to swing the driven mirrors 12a and 12b so that the treating laser beam is irradiated to scan the treatment area.

On completion of one scanning operation, the scanner controller 23 operates on the drive motor 61 to retract the driven mirror 60 to the position P_{ES}. The diseased part immediately after the laser irradiation is imaged by the CCD camera 62 and displayed on the display screen 41. The operator can immediately and easily observe on the display screen 41 the result of laser irradiation imaged at the same view point (from above the diseased part in this embodiment) as of the image before the irradiation has taken place without the need of moving the hand piece unit 3 out of the area of diseased part. Accordingly, the operator can readily proceed to the recurring laser irradiation when necessary without expending the time and labor for the repositioning of laser irradiation area to the diseased part.

Although in this embodiment the driven mirror 60 is turned to interrupt the light path of the CCD camera 62 at laser irradiation so that it serves as a shutter against the laser beam, an alternative scheme is the use of a beam splitter which reflects and transmits the treating laser beam and visible aiming light beam, respectively, and a filter which blocks the treating laser beam so that the CCD camera 62 images simultaneously the diseased part and the aiming light projected onto the diseased part.

Although the image of diseased part displayed on the display screen 41 can readily be enlarged or reduced based on the image processing technique, an alternative scheme is to enlarge or reduce the image of diseased part in optical manner by the provision of a zooming optical system on the light path of the CCD camera 62.

Although the third embodiment uses a LCD touch-panel display screen, an alternative design is the use of a simple LCD display screen, with additional keys being attached on the control panel 7 or the existing keys being assigned to additional functions for the substitution for the icons of keys provided on the LCD touch-panel display screen. Alternatively, the whole control panel 7 may be formed of a LCD touch-panel display screen, in which case all icons of keys may be displayed at once or some icons of keys may be displayed selectively depending on the preceding key action taken by the operator.

Besides the above-mentioned variants of the present invention, other modifications may be made within the scope of the appended claims.

According to the present invention as described above, it becomes possible to optimize easily the scanning area of the treating laser beam to meet the shape of treatment area, thereby performing the proper laser treatment. The automatic determination of the scanning area to meet the shape of treatment area by the inventive apparatus reduces the operator's work significantly. The efficient laser beam scanning operation relieves the operator of many foot switch depressing actions.

## Claims

1. A laser treatment apparatus for irradiating a treatment area with a laser beam comprising:
a laser source (21) for emitting said laser beam,
laser beam scanning means (12a, 12b, 17a, 17b, 60, 61, 13) for scanning the treatment area with the laser beam; scanning area determining means (20, 23, 62) for determining a laser beam scanning area corresponding to the treatment area to be scanned with the laser beam by said laser beam scanning means;
memory means (25) connected to said scanning area determining means for storing information on the laser beam scanning area determined by said scanning area determining means; and
control means (20, 23) connected to said laser source, said laser beam scanning means and said memory means for controlling said laser beam scanning means based on the information on the laser beam scanning area read out of said memory means (25) to scan said treatment area;
**characterized in that**
said scanning area determining means includes:
imaging means (62) for imaging photo-electrically an area including at least the treatment area;
treatment area detecting means (20, 23, 62) for detecting the treatment area based on an image produced by said imaging means; and
means for determining the laser beam scanning area based on the result of detection by said treatment area detecting means.

2. A laser treatment apparatus according to claim 1,
wherein:
said treatment area detecting means detects said treatment area from the image signal.

3. A laser treatment apparatus according to claim 1 or 2,
further including display means (41) for displaying an image; and
display control means for creating a figure of the laser beam scanning area based on the information on the laser beam scanning area determining by said scanning area determining means, and
superimposing the figure of the scanning area and the image corresponding to the image signal generated by said imaging means displayed on said display means.

4. A laser treatment apparatus according to one of claims 1 and 3, wherein
said memory means (25) furthermore can store information on a scanning area having a predetermined shape other than the scanning area determined by said scanning area determining means, and/or
wherein said memory means can store information on a plurality of scanning areas determined by said scanning area determining means, and
said apparatus further including selecting means for selecting one of the scanning areas stored in said memory means.

5. A laser treatment apparatus according to one of claims 1 to 4, further including
trigger signal input means (8) connected to said control means for generating a trigger signal for starting the emission of the laser beam of said laser source and the operation of said laser beam scanning means; wherein said control means includes mode selection means for, when said trigger signal input means is generating the trigger signal continuously, selecting the repeat mode in which said control means controls said laser source and said laser beam scanning means to repeatedly scan the treatment area.

6. A laser treatment apparatus according to claim 5, further including:
halt time interval setting means for, when said mode selection means selects the repeat mode, setting a time interval of halting of the repetitive operations, and
scanning end detection means for detecting the end of one laser beam scanning operation by said laser beam scanning means,
said control means controlling the repetitive operations of said laser source and said laser beam scanning means based on the halt time interval set by said halt time interval setting means and the result of detection by said scanning end detection means such that each of the repetitive operations is separated from the next one by the halt time interval.

## Patentansprüche

1. Laserbehandlungsvorrichtung zum Bestrahlen einer Behandlungsfläche mit einem Laserstrahl mit:
einer Laserquelle (21) zum Aussenden des Laserstrahls,
einer Laserstrahlabtasteinrichtung (12a, 12b, 17a, 17b, 60, 61, 13) zum Abtasten der Behandlungsfläche mit dem Laserstrahl;
einer Abtastflächenbestimmungseinrichtung (20, 23, 62) zum Bestimmen einer Abtastfläche für den Laserstrahl entsprechend
der mit dem Laserstrahl durch die Laserstrahlabtasteinrichtung abzutastenden Behandlungsfläche;
einer Speichereinrichtung (25), die mit der Abtastflächenbestimmungseinrichtung verbunden ist, zum Speichern von Information über die Laserstrahlabtastfläche, die durch die Abtastflächenbestimmungseinrichtung bestimmt worden ist; und
einer Steuereinrichtung (20, 23), die mit der Laserquelle, der Laserstrahlabtasteinrichtung und der Speichereinrichtung verbunden ist, zum Steuern der Laserstrahlabtasteinrichtung auf der Grundlage der Information über die Laserstrahlabtastfläche, die aus der Speichereinrichtung (25) ausgelesen worden ist,zum Abtasten der Behandlungsfläche;
**dadurch gekennzeichnet, daß**
die Abtastflächenbestimmungseinrichtung aufweist:
eine Abbildungseinrichtung (62) zum photoelektrisch Abbilden einer Fläche, die wenigstens die Behandlungsfläche beinhaltet; einer Behandlungsflächenerfassungseinrichtung (20, 23, 62) zum Erfassen der Behandlungsfläche auf der Grundlage eines Bildes, das durch die Abbildungseinrichtung erzeugt worden ist; und
einer Einrichtung zum Bestimmen der Laserstrahlabtastfläche auf der Grundlage des Ergebnisses der Erfassung durch die Behandlungsflächenerfassungseinrichtung.

2. Laserbehandlungsvorrichtung nach Anspruch 1, wobei die Behandlungsflächenerfassungseinrichtung die Behandlungsfläche von dem Bildsignal erfaßt.

3. Laserbehandlungseinrichtung nach Anspruch 1 oder 2, die
weiter eine Anzeigeeinrichtung (41) zum Anzeigen eines Bildes; und
eine Anzeigesteuereinrichtung zum Erzeugen einer Figur der Laserstrahlabtastfläche auf der Grundlage der Information über die Laserstrahlabtastfläche, bestimmt durch die Abtastflächenbestimmungseinrichtung, und
zum Überlagern der Figur der Abtastfläche und des Bildes entsprechend dem Bildsignal, das durch die Abbildungseinrichtung erzeugt worden ist und auf der Anzeigevorrichtung gezeigt wird,
aufweist.

4. Laserbehandlungsvorrichtung nach einem der Ansprüche 1 und 3, wobei
die Speichereinrichtung (25) ferner Information über eine Abtastfläche, die eine vorbestimmte Form aufweist, die anders ist als die Abtastfläche, die durch die Abtastflächenbestimmungseinrichtung bestimmt worden ist, speichern kann und/oder
wobei die Speichereinrichtung Information über eine Mehrzahl von Abtastflächen, die durch die Abtastflächenbestimmungseinrichtung bestimmt worden sind, speichern kann und
die Vorrichtung ferner eine Auswahleinrichtung aufweist zum Auswählen einer der Abtastflächen, die in der Speichereinrichtung gespeichert sind.

5. Laserbehandlungsvorrichtung nach einem der Ansprüche 1 bis 4, weiter mit
einer Triggersignaleingabeeinrichtung (8), die mit der Steuereinrichtung verbunden ist, zum Erzeugen eines Triggersignals zum Starten der Aussendung des Laserstrahls der Laserquelle und des Betriebs der Laserstrahlabtasteinrichtung; wobei die Steuereinrichtung eine Modusauswahleinrichtung aufweist zum Auswählen des Wiederholmodus, wenn die Triggersignaleingabeeinrichtung das Triggersignal kontinuierlich erzeugt, in welchem die Steuereinrichtung die Laserquelle und die Laserstrahlabtasteinrichtung steuert zum wiederholten Abtasten der Abtastfläche.

6. Laserbehandlungsvorrichtung nach Anspruch 5, weiter mit:
einer Haltezeitintervalleinstelleinrichtung zum Einstellen eines Zeitintervalls des Anhaltens der wiederholten Vorgänge, wenn die Modusauswahleinrichtung den Wiederholmodus auswählt, und
einer Abtastendeerfassungseinrichtung zum Erfassen des Endes des Laserstrahlabtastvorgangs durch die Laserstrahlabtasteinrichtung,
wobei die Steuereinrichtung die wiederholten Vorgänge der Laserquelle und der Laserstrahlabtasteinrichtung auf der Grundlage des Haltezeitintervalls, das durch die Haltezeitintervalleinstelleinrichtung eingestellt worden ist und des Ergebnisses der Erfassung durch die Abtastendeerfassungseinrichtung derart steuert, daß jeder der wiederholten Vorgänge von dem nächsten durch das Haltezeitintervall getrennt ist.

## Revendications

1. Appareil de traitement laser pour irradier une zone de traitement avec un faisceau laser comprenant :
une source laser (21) pour émettre ledit faisceau laser,
un moyen de balayage de faisceau laser (12a, 12b, 17a, 17b, 60, 61, 13) pour balayer la zone de traitement avec le faisceau laser ;
un moyen de détermination de zone de balayage(20, 23, 62) pour déterminer une zone de balayage du faisceau laser correspondant à la zone de traitement à balayer avec le faisceau laser par ledit moyen de balayage de faisceau laser ;
un moyen de mémoire (25) relié audit moyen de détermination de zone de balayage pour mémoriser les informations concernant la zone de balayage du faisceau laser déterminées par ledit moyen de détermination de zone de balayage ; et
un moyen de commande (20, 23) relié à ladite source laser, audit moyen de balayage de faisceau laser et audit moyen de mémoire pour commander ledit moyen de balayage du faisceau laser basé sur les informations concernant la zone de balayage du faisceau laser extraites dudit moyen de mémoire (25) pour balayer ladite zone de traitement ;
**caractérisé en ce que**
ledit moyen de détermination de zone de balayage inclut :
un moyen de formation d'image (62) pour former photoélectriquement en image une zone incluant au moins la zone de traitement ;
un moyen de détection de zone de traitement (20, 23, 62) pour détecter la zone de traitement basé sur une image produite par ledit moyen de formation d'image ; et
un moyen pour déterminer la zone de balayage du faisceau laser sur la base du résultat de la détection par ledit moyen de détection de zone de traitement.

2. Appareil de traitement laser selon la revendication 1, dans lequel :
ledit moyen de détection de zone de traitement détecte ladite zone de traitement à partir du signal d'image,

3. Appareil de traitement laser selon la revendication 1 ou 2, incluant, en outre, un moyen d'affichage (41) pour afficher une image ; et
un moyen de commande d'affichage pour créer une figure de la zone de balayage du faisccau laser sur la base des informations de la zone de balayage du faisceau laser déterminées par ledit moyen de determination de zone de balayage, et
pour superposer la figure de la zone de balayage et l'image correspondant au signal d'image généré par ledit moyen de formation d'image affiché sur ledit moyen d'affichage.

4. Appareil de traitement laser selon l'une quelconque des revendications 1 et 3, dans lequel
ledit moyen de mémoire (25) peut, en outre, mémoriser des informations concernant une zone de balayage ayant une forme prédéterminée autre que la zone de balayage déterminée par ledit moyen de détermination de zone de balayage, et/ou
dans lequel ledit moyen de mémoire peut mémoriser des informations concernant une pluralité de zones de balayage déterminées par ledit moyen de détermination de zone de balayage, et
ledit appareil incluant, de plus, un moyen de sélection pour sélectionner une des zones de balayage mémorisées dans ledit moyen de mémoire.

5. Appareil de traitement laser selon l'une -quelconque des revendications 1 à 4, incluant de plus
un moyen d'entrée de signal de déclenchement (8) relié audit moyen de commande pour générer un signal de déclenchement pour commencer l'émission du faisceau laser de ladite source laser et l'opération dudit moyen de balayage du faisccau laser ; dans lequel ledit moyen de commande inclut un moyen de sélection de mode pour lorsque ledit moyen d'entrée de signal de déclenchement génère continuellement le signal de déclenchement, sélectionner le mode répétitif dans lequel ledit moyen de commande commande ladite source laser et ledit moyen de balayage du faisceau laser pour balayer de manière répétée la zone de traitement.

6. Appareil de traitement laser selon la revendication 5, incluant en outre :
un moyen de réglage d'intervalle de temps d'arrêt pour, lorsque ledit moyen de sélection de mode sélectionne le mode répétitif, régler un intervalle de temps d'arrêt des opérations répétitives, et
un moyen de détection de fin de balayage pour détecter la fin d'une opération de balayage du faisceau laser par ledit moyen de balayage du faisceau laser,
ledit moyen de commande commandant les opérations répétitives de ladite source laser et dudit moyen de balayage du faisceau laser sur la base de l'intervalle de temps d'arrêt réglé par ledit moyen de réglage d'intervalle de temps d'arrêt et du résultat de la détection par ledit moyen de détection de fin de balayage de sorte que chacune des opérations répétitives est séparée de la suivante par l'intervalle de temps d'arrêt.
